# EUROPÄISCHE PATENTANMELDUNG

(11) **EP 2 535 421 A1**
(43) Veröffentlichungstag der Anmeldung: **19.12.2012**
(21) Anmeldenummer: 12184266.0
(22) Anmeldetag: 31.03.2008
(51) Int. Cl.: C12P 19/02, C12P 19/14, C12P 13/08

(54) **Verfahren zur enzymatischen Hydrolyse chemisch vorbehandelter Lignocellulose**

(30) Priorität: 26.04.2007 DE 102007019643
(62) Teilanmeldung aus: 08103271.6
(71) Anmelder: Evonik Degussa GmbH, 45128 Essen (DE)
(72) Erfinder: Villela Filho, Murillo, 46244 Bottrop-Kirchhellen (DE); Mai, Mirjam, 14469 Potsdam (DE); Karau, Andreas, 63571 Gelnhausen (DE)

(57) **Zusammenfassung**

Die Erfindung betrifft in Fermentationsprozessen verwendbare und durch enzymatische Hydrolyse von Lignocellulose enthaltenden Materialien gewonnene Lösungen oder Suspensionen, Verfahren zu deren Herstellung und ihre Verwendung bei der Fermentation zur Herstellung von organischen Zielsubstanzen.

## Beschreibung

Die Erfindung betrifft in Fermentationsprozessen verwendbare und durch enzymatische Hydrolyse von Lignocellulose enthaltenden Materialien gewonnene Lösungen oder Suspensionen, Verfahren zu deren Herstellung und ihre Verwendung bei der Fermentation zur Herstellung von organischen Zielsubstanzen.

### Stand der Technik

Viele dieser Substanzen werden mittlerweile alternativ zu klassischen Verfahren durch biotechnologische Prozesse gewonnen. Mikroorganismen setzten dabei die zugeführten Kohlenstoffquellen (C-Quelle) in Fermentationsprozessen zu den gewünschten Produkten um. Die Kosten der Kohlenstoffquelle beschränken jedoch die Margen in diesem Geschäft. Der größte Posten in den Herstellungskosten von beispielsweise L-Lysin ist die C-Quelle. Der Preis, der hierfür am häufigsten eingesetzten Zucker wie Glucose oder auch Saccharose, unterliegt starken Schwankungen und hat einen schwerwiegenden Einfluss auf die Wirtschaftlichkeit eines Fermentationsprozesses vor allem bei den niedrigpreisigen Massenprodukten wie Mononatriumglutamat, L-Lysine-HCl und L-Threonin, deren Markt stark vom Wettbewerb bestimmt wird (M. Ikeda, Advances in Biochimical Engineering / Biotechnology, Vol. 79 (2003) 2-35).

Es gibt daher zahlreiche Versuche, aus günstigen Nachwachsenden Rohstoffen geeignete Kohlenstoffquellen für die Fermentation zu gewinnen.

Neben Stärke ist auch Cellulose aus Glucosemonomeren aufgebaut und kann durch entsprechende Hydrolyse in diese gespalten werden. Hierzu sind andere Enzyme als beim Stärkeabbau notwendig, da es sich um β-1,4-verknüpfte Monomere handelt. Cellulose ist grundlegender Zellbestandteil aller Pflanzen und somit das am meisten verbreitete nachwachsende Biopolymer der Welt.

Lignocellulosehaltiges Stroh fällt in der Landwirtschaft als Abfall an und stellt einen kostengünstigeren Rohstoff für Hydrolysen dar als speziell zu diesem Zweck angebaute, stärkehaltige Pflanzen. Allerdings bestehen pflanzliche Zellwände aus einem netzwerkartigen Geflecht von Cellulose, Hemicellulose und Lignin. Letztere schirmen die Cellulose vor einem mikrobiellen Angriff ab, was in der Natur für die Stabilität der Pflanzen wichtig ist. Auch die teilkristalline Struktur der Cellulose erschwert die enzymatische Abbaubarkeit. Diese ist zwar prinzipiell möglich, aber nur mit schlechten Ausbeuten zu erreichen. Für die industrielle Nutzung ist eine hohe Hydrolyserate mit großer Glucoseausbeute erwünscht. Zudem soll das Hydrolysat als C-Quelle in Fermentationen zur Gewinnung von Feinchemikalien oder Ethanol eingesetzt werden können.

Ein Überblick über den gegenwärtigen Stand der Entwicklung auf dem Gebiet der Nutzung nachwachsender Rohstoffe in der industriellen Chemikalienproduktion findet sich bei R. Busch et al. (Chem. Ing. Tech. 78 (2006) 219-228). Danach gelingt die Überführung von Cellulose und Hemicellulose in fermentierbare Produkte durch die Einwirkung von Säuren und hydrolytisch wirksamen Enzymen.

### Problemstellung

Zur Gewinnung von fermentierbaren Hydrolysaten aus lignocellulosehaltigen Rohstoffen müssen diese Rohstoffe aufgrund ihrer netzwerkartigen Struktur vor der Hydrolyse physikalisch und chemisch vorbehandelt werden. Unter lignocellulosehaltigen Rohstoffen sind zu verstehen:
● Holz von Laub- und Nadelbäumen
● Stroh von z.B. Mais, Roggen, Weizen, Hafer, Gerste, Sorghum, Raps, Reis
● Bagasse.

Um eine größere Oberfläche für die Einwirkung von Chemikalien und Enzymen bereitstellen zu können, werden die Rohstoffe mit Mühlen zerkleinert. Wünschenswert sind Partikelgrößen zwischen 0,5-10 mm, vorzugsweise zwischen 0,5-5 mm, insbesondere zwischen 1-3 mm.

Durch die chemische Vorbehandlung werden die enthaltenen Hemicellulosen in Lösung gebracht und das vernetzte aromatische Gerüst des Lignins aufgebrochen. Die teilkristalline Cellulosestruktur wird aufgelöst und für den Angriff von Enzymen zugänglicher gemacht.

Aufgabe der Erfindung ist es, ein durch Hydrolyse entstandenes Produkt bereitzustellen, das als C-Quelle für Fermentationen zur Herstellung von organischen Zielsubstanzen geeignet ist.

Darunter fallen organische Substanzen mit mindestens einem N-Atom und/oder organische Substanzen mit mindestens drei C-Atomen. Bekannte Beispiele für gewünschte Verbindungen sind Alkohole, L-Aminosäuren, Vitamine, Antibiotika, Nukleinsäuren, Proteine, Enzyme und organische Säuren. Zu den L-Aminosäuren zählen insbesondere L-Lysin, L-Homoserin, L-Threonin, L-Valin, L-Isoleucin, L-Prolin, L-Methionin und L-Tryptophan.

### Beschreibung der Erfindung

Gegenstand der Erfindung ist ein Verfahren zur Herstellung von zuckerhaltigen Hydrolysaten von Lignocellulose enthaltenden Materialien, umfassend
a) die Vorbehandlung dieser Materialien mit Peroxodisulfaten, insbesondere Kaliumperoxodisulfat oder Ammoniumperoxodisulfat in Gegenwart von Wasser, und
b) nach der Abtrennung der wässrigen Phase und Waschen des entstandenen Produkts dessen Behandlung mit einem für die Hydrolyse geeigneten Enzym in Gegenwart von Wasser.

Bei diesem Verfahren verwendet man Lignocellulose enthaltende Materialien, Holz von Laub- und Nadelbäumen oder setzt Stroh ein, ausgewählt aus den Pflanzenarten Mais, Roggen, Weizen, Hafer, Gerste, Sorghum, Raps oder Reis, Bagasse.

Um eine größere Angriffsfläche für die eingesetzten Verbindungen zu erhalten, zerkleinert oder zermahlt man die Ausgangsmaterialien nach den bekannten Verfahren.

Mit dem Begriff zuckerhaltige Hydrolysate sind Gehalte folgender Zucker erfasst: Hexosen wie z.B. Glucose, Mannose, Galactose und deren Derivaten, wie z. B. Gluconsäure, Glucarsäure oder Glucuronsäure oder Pentosen wie Xylose oder Arabinose und deren Derivaten.

Der pH-Wert der Vorbehandlung liegt zwischen 1 und 14 und wird bevorzugt in Abhängigkeit von den eingesetzten Verbindungen ausgewählt. Die enzymatische Hydrolyse wird im Allgemeinen bei pH-Wert im Bereich von 4 bis 7, bevorzugt von 4,5 bis 5,5 durchgeführt.

### Als Enzyme setzt man im Allgemeinen

Cellulasen z.B. Spezyme Cp von Novozyme (kommerziell erhältlich) ein.

Zur Vorbehandlung im sauren Milieu wird verdünnte Schwefelsäure mit einer Konzentration von 0.5-5%, vorzugsweise 1-2%, vorzugsweise 1-1,5% verwendet. Gew.-% bezieht sich auf Schwefelsäuregehalt (g/ 100g). Die saure Strohsuspension wird im Autoklaven unter Ausbildung von Eigendruck bei einer Temperatur von 80-150°C, vorzugsweise 90-140°C, vorzugsweise 100-130°C für 90 Minuten gerührt. Diese Bedingungen sind entsprechend auf die Behandlung mit KOH zu übertragen.

Als Oxidationsmittel können Alkaliperoxodisulfate, insbesondere Kaliumperoxodisulfat und Kaliumperoxid in Konzentrationen von 0.5-5-% eingesetzt werden.

Das Stroh wird anschließend abgetrennt und bevorzugt mehrfach gewaschen, um die gelösten toxischen Stoffe zu entfernen. Das gewaschene Stroh wird dann für die Hydrolyse verwendet und in Form einer Suspension vorgelegt. Die verwendten Enzyme sind aus dem Stand der Technik bekannt (WO 2004/081185). Ein Cellulasekomplex spaltet in der anschließenden enzymatischen Hydrolyse die Celluloseketten in kleinere Fragmente bis hin zu Glucosemonomeren. Die Menge an Enzym(Protein) sollte 0.1-5 g/mL, vorzugsweise 0.1-3 g/mL betragen und in Abständen von wenigen Stunden in kleineren Portionen zugegeben werden. Die optimale Menge hängt von der in der Vorbehandlung benutzten Verbindung ab. Die Hydrolyse wird bei einer Temperatur von 30-70°C, vorzugsweise 40-65°C, vorzugsweise 50-60°C über eine Dauer von 24 h durchgeführt. Der pH-Wert sollte 3-7, vorzugsweise 4-6, vorzugsweise 4,5-5,5 betragen. Anschließend trennt man bevorzugt die noch vorhandenen Feststoffe ab.

Die Glucose enthaltende Lösung wird nach entsprechender Aufkonzentrierung, die bevorzugt im Vakuum erfolgt, in Fermentationen erfolgreich als C-Quelle eingesetzt.

Das erfindungsgemäße Verfahren wurde durch die enzymatische Hydrolyse von fein gemahlenen Mais-, Roggen- und Weizenstroh (Durchschnittsgröße 1-3 mm) mit einem Cellulase-Enzym-Komplex (Novozymes, Dänemark) experimentell belegt. Das Hydrolysat wurde im Vakuumrotationsverdampfer aufkonzentriert und anschließend durch Autoklavieren sterilisiert.

Die Eignung derartig hergestellter Weizen- und Maisstrohhydrolysate als Kohlenstoffquelle für die fermentative Herstellung von Feinchemikalien wurde am Beispiel der L-Lysinherstellung mit dem Stamm *Brevibacterium flavum* DM1730 (Georgi et al. 2005) in Schüttelkolbenexperimenten unter Kontrolle der Stoffwechselaktivität demonstriert. Dabei zeigte sich zusätzlich überraschenderweise, dass die Experimente mit Hydrolysat teilweise zu deutlich höherer Produktbildung führten, als das Kontrollexperiment mit Standardglucoselösung.

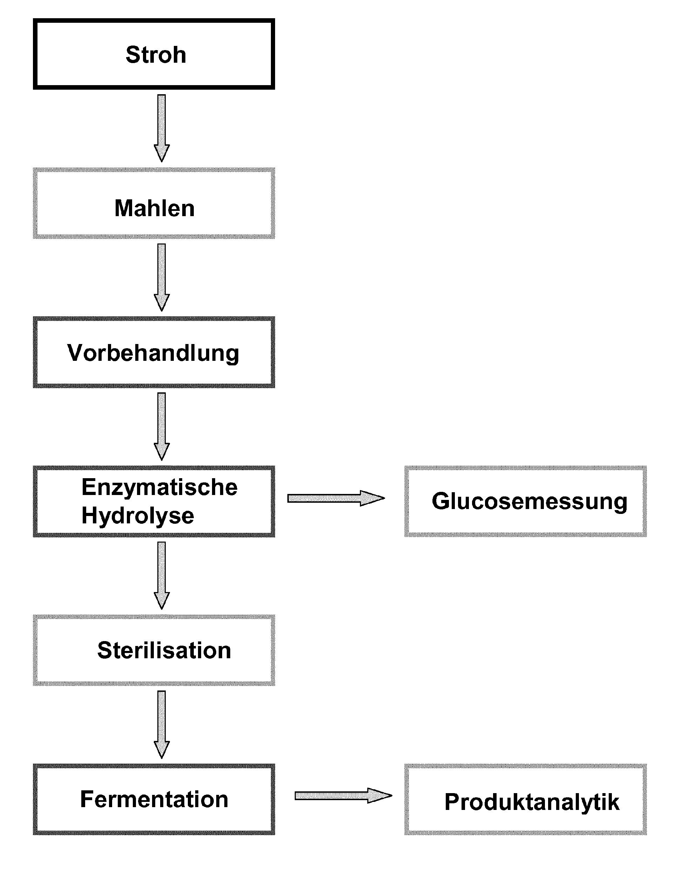

Gegenstand der Erfindung ist daher ebenso ein Verfahren, gemäß den Ansprüchen 1 bis 3, an das sich anschließt:
ein Verfahren zur Herstellung einer organischen Zielsubstanz mit mindestens 3 C-Atomen oder mindestens 2 C-Atomen und einem N-Atom durch Fermentation von diese Substanzen herstellenden Mikroorganismen, wobei man das erfindungsgemäß hergestellte zuckerhaltige Hydrolysat als C-Quelle einsetzt. Diese kann gegebenenfalls durch weitere C-Quellen ergänzt werden
Im Anschluß an die Fermentation wird die Zielsubstanz bevorzugt isoliert, gegebenenfalls mit der Gesamtmenge oder Anteilen der Biomasse. Die Fermentationsbrühe wird im Allgemeinen zuvor schonend, d. h. ohne Zersetzung von Bestandteilen eingeengt. In Abb. 1 ist das Ablaufschema des gesamten Prozesses wiedergegeben.

Nach dem erfindungsgemäßen Verfahren werden insbesondere Zielprodukte hergestellt, die ausgewählt sind aus der Gruppe: Mono-, Di- und Tricarbonsäuren mit 3 bis 10 C-Atomen, proteinogene und nicht-proteinogene L-Aminosäuren, gesättigten und ungesättigten Fettsäuren; Diole mit 3 bis 8 C-Atomen, höherwertige Alkohole mit 3 oder mehr Hydroxylgruppen, längerkettige Alkohole mit wenigstens 4 C-Atomen, Vitamine, Provitamine, Ketone mit 3 bis 10 C-Atomen.

Die Zielprodukte werden insbesondere aus der Gruppe, bestehend aus L-Aminosäuren und Vitaminen wie insbesondere L-Lysin, L-Methionin, L-Threonin, L-Prolin, L-Isoleucin, L-Homoserin, L-Valin, Pantothensäure und Riboflavin, sowie Propionsäure, Propandiol, Butanol und Aceton ausgewählt.

Die Mikroorganismen werden ausgewählt aus solchen, die insbesondere L-Aminosäuren oder Vitamine produzierenden, insbesondere aus L-Lysin, L-Methionin, L-Threonin, L-Prolin, L-Isoleucin, L-Homoserin, L-Valin, Pantothensäure Riboflavin, Propionsäure, Propandiol, Butanol, Aceton und Trehalose produzierenden Mikroorganismen.

Unter produzierenden Mikroorganismen sind dabei solche zu verstehen, die die gewünschten Zielprodukte im Vergleich zum Wildtyp in erhöhtem Umfang produzieren und gegebenenfalls ausscheiden.

Die Fermentation der Mikroorganismen, kann kontinuierlich - wie beispielsweise in der PCT/EP 2004/008882 beschrieben - oder diskontinuierlich im batch-Verfahren (Satzkultivierung) oder im fed batch (Zulaufverfahren) oder repeated fed batch Verfahren (repetitives Zulaufverfahren) zur Produktion von der Zielsubstanzen durchgeführt werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können neben durch Hydrolyse erzeugten Zuckern weitere Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniak, Ammoniumsulfat, Ammoniumphosphat, Ammoniumcarbonat und Ammoniumnitrat, bevorzugt Ammoniak oder Ammoniumsulfat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe werden zur Kultur in Form eines einmaligen Ansatzes hinzugegeben oder in geeigneter Weise während der Kultivierung zugefüttert.

Zur pH-Kontrolle der Kultur werden basische Verbindungen wie Natriumhydroxid, Kaliumhydroxid, Ammoniak beziehungsweise Ammoniakwasser bevorzugt Ammoniak oder Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium gegebenenfalls geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20 °C bis 45 °C und vorzugsweise bei 25 °C bis 40 °C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Beispiele für geeignete Fermentationsmedien finden sich unter anderem in den Patentschriften US 5,770,409, US 5,840,551 und US 5,990,350, US 5,275,940 oder US 4,275,157. Weitere Beispiele für Fermentationsmedien finden sich bei Ozaki und Shiio (Agricultural and Biological Chemistry 47(7), 1569-1576, 1983) und Shiio et al. (Agricultural and Biological Chemistry 48(6), 1551-1558, 1984).

In einer bevorzugten Ausführungsform handelt es sich bei dem durch Fermentation hergestellten Produkt um eine L-Aminosäure, insbesondere um L-Lysin. Zur Durchführung der Fermentation können hier analoge Bedingungen und Vorgehensweisen angewendet werden, wie sie z. B. bei Pfefferle et al., Advances in Biochemical Engingeering/Biotechnology, Vol. 79 (2003) 60-112 und in der US 3,708,395 beschrieben werden.

Erfindungsgemäß eingesetzte Mikroorganismen werden bevorzugt ausgewählt unter den Gattungen Corynebacterium, Bacillus, Escherichia, Aspergillus, Lactobacillus, insbesondere unter Stämmen von Corynebacterium glutamicum, Bacillus subtillis, Escherichia coli oder Aspergillus niger.

Es sind viele chemische Methoden zur Vorbehandlung von Cellulose zur Steigerung der Hydrolysate bekannt. Jedoch eignen sich nicht alle Hydrolysate als C-Quelle für Fermentation. Besonders geeignet sind die erfindungsgemäße Vorbehandlung mit Kaliumperoxodisulfat.

Es konnte ebenfalls gezeigt werden, dass dies nicht für die alkalische Vorbehandlung mit Natriumhydroxid (durchgeführt nach Varga et al.) Varga et al., Applied Biochemistry and Biotechnology, vol. 98-100 (2002) 75-86 und für die oxidativen Vorbehandlungen mit Peressigsäure, Wasserstoffperoxid oder Natriumhypochlorit (durchgeführt nach WO 2004/081185), und die kombinierte Anwendung von Natriumhydroxid und schwefelsäure (2-Schritt, nach Varga et al.) zutrifft. Möglicherweise entstehen toxische Nebenprodukte, die das Wachstum von Mikroorganismen verhindern, so dass auch keine Produktbildung stattfindet.

### Experimenteller Teil

Es konnte gezeigt werden, dass nur ausgwählte Vorbehandlungsmethoden wie der Einsatz von Schwefelsäure oder bestimmter Oxidationsmittel zu Hydrolysaten führen, die in Fermentationsprozessen als C-Quelle eingesetzt werden können.

### Beispiele

### 1. Vorbehandlung

### 1.1 Schwefelsäure (nicht zur Erfindung gehörend)

Aus konzentrierter Schwefelsäure (Oleum, 96 %) wird durch Verdünnen eine 1.2 %ige Lösung hergestellt. 10 g gemahlenes Stroh werden in einen Tantal-Autoklaven gefüllt und mit 500 mL der verdünnten Säure versetzt. Unter Ausbildung von Eigendruck wird der Autoklav auf 120 ° C geheizt. Bei dieser Temperatur wird die Strohsuspension für 90 min. gerührt und anschließend abgekühlt. Der Vorgang wird wiederholt, so dass 1 L der vorbehandelten Strohsuspension für die Hydrolyse zur Verfügung steht.

### 1.2 Oxidationsmittel

20 g Stroh werden in etwa 800 mL VE-Wasser vollentsalztes Wasser suspendiert. Das Oxidationsmittel wird in etwa 200 mL VE-Wasser gelöst und langsam über einen Tropftrichter zur Strohsuspension gegeben. Angaben der genauen Mengen sind aus Tabelle 1 zu entnehmen. Die Zutropfgeschwindigkeit sollte etwa 1 Tropfen pro Minute betragen, um zu starken Temperaturanstieg zu vermeiden. Sobald die Zugabe beendet ist, wird das Thermostat eingeschaltet und die Suspension auf 80 ° C geheizt. Unter Rühren (150 rpm) wird die Suspension für 24 h bei dieser Temperatur gehalten. Anschließend wird das Vorhandensein des Oxidationsmittels mit Peroxidstäbchen oder Kaliumiodid-Stärke-Papier getestet und dieses gegebenenfalls mit katalytischen Mengen Eisen(II)sulfat zerstört.

**Tabelle 1: Eingesetzte Mengen an Oxidationsmittel**

| | KO₂ | K₂S₂O₈ | KOH |
|---|---|---|---|
| VE-Wasser zum Suspendieren | 800 mL | 800mL | 1000 mL |
| Oxidationsmittel | 5 g | 10 g | 4 g |
| VE-Wasser zum Lösen | 200 mL | 200 mL | - |

### 2. Nicht erfindungsgemäße Vorbehandlung

### 2.1 Alkalisch (nach Varga et al.)

Aus NaOH-Plätzchen wird 1 L einer 1 %igen Lösung hergestellt. 10 g gemahlenes Stroh werden in einen 1-L-Hastelloy-Autoklaven gefüllt und mit 500 mL der verdünnten Natronlauge versetzt. Unter Ausbildung von Eigendruck wird der Autoklav auf 120°C geheizt. Bei dieser Temperatur wird die Strohsuspension für 60 min. gerührt und anschließend abgekühlt. Der Vorgang wird wiederholt, so dass 1 L der vorbehandelten Strohsuspension für die Hydrolyse zur Verfügung steht.

### 2.2 2-Schritt (nach Varga et al.)

20 g Stroh werden bei Raumtemperatur für 24 h in 1 %iger Natronlauge unter Rühren (150 rpm) suspendiert. Anschließend wird das Stroh mittels Vakuumpumpe über einen Filter abgenutscht und mehrmals mit VE-Wasser gewaschen. Das Stroh wird im Autoklaven in 1 %iger Schwefelsäure suspendiert und für 1 h bei 120°C unter Ausbildung von Eigendruck gerührt.

### 3. Oxidationsmittel (nach WO 2004/081185)

Wie unter erfindungsgemäße Vorbehandlung, aber andere Oxidationsmittel (Tab. 2)

**Tabelle 2: Eingesetzte Mengen an Oxidationsmittel**

| | NaOCl | CH₃COOOH | H₂O₂ |
|---|---|---|---|
| VE-Wasser zum Suspendieren | 750 mL | 800 mL | - |
| Oxidationsmittel | 100 mL | 26 mL | 6 mL |
| VE-Wasser zum Lösen | 150 mL | 174 mL | 994 mL |

### 4. Enzymatische Hydrolyse

Die wie unter 1 und 2 beschriebenen vorbehandelte Strohsuspension wird in einen 6-L-Fermenter gefüllt und mit 10 mM Citratpuffer und 10 M Natronlauge bzw. konzentrierter Schwefelsäure auf pH 5.0 eingestellt. Die Suspension wird mit 100 rpm gerührt, auf 60 C geheizt und dann werden 2 mL (0.133 g/mL Protein) Enzympräparation Spezyme Cp (Novozymes, Dänemark) zugegeben. Die Hydrolyse wird über 24 h durch regelmäßige Messungen der Glucosekonzentration verfolgt. Danach wird die Hydrolyse durch Abkühlen beendet.

### 5. Fermentation

### 5.1 Standard

Ein CgXII-Standardmedium mit reiner Glucose wird nach dem Rezept von Keilhauer et al. (J. Bacteriol., Vol. 175 (1993)5595) mit reduziertem Glucosegehalt hergestellt (s. Tab. 3)und im Autoklav sterilisiert. Die stickstoffhaltigen Salze werden separat in VE-Wasser gelöst und im Autoklav sterilisiert. Biotin- und Protokatechusäurestammlösung werden nicht thermisch sterilisiert, sondern frisch angesetzt und durch einen Sterilfilter (Porenweite 0.2 µm) ins Medium gegeben.

### 5.2 Hydrolysate

Aus den Hydrolysaten werden die Medien nach dem Rezept von Keilhauer et al. für CgXII-Medium hergestellt. Als C-Quelle dienen die aufkonzentrierten Hydrolysate, in denen die Nährsalze gelöst werden. Zum Vergleich wird ein Standardmedium mit reiner Glucose hergestellt. Die Glucosekonzentration in allen Medien wird so eingestellt, dass alle vor der Sterilisation dieselbe Konzentration aufweisen. Nach der Sterelisation können sie sich jedoch wieder unterscheiden.

**Tabelle 3: CgXII-Medium (nach Keilhauer et al. 1993, modifiziert**

| Substanz | Endkonzentration | Volumen [mL] | Einwaage | Entnahme Stammlösung [mL] | pH |
|---|---|---|---|---|---|
| (NH₄)₂SO₄ | 20 g/L | 150 | 3 g | 0,15 | 7 |
| Harnstoff | 5 g/L | 150 | 0,75 | 0,15 | 7 |
| KH₂PO₄ | 1 g/L | 150 | 0,15 | 0,15 | 7 |
| K₂HPO₄ | 1 g/L | 150 | 0,15 | 0,15 | 7 |
| MgSO₄ × 7H₂O | 0,25 g/L | 150 | 0,0375 | 0,15 | 7 |
| MOPS | 42 g/L | 150 | 6,3 | 0,15 | 7 |
| CaCl₂ | 10 mg/L | 20 | 0,2 mL | 0,15 | 7 |
| FeSO₄ × 7H₂O | 10 mg/L | 50 | 0,5 | 0,15 | 1 |
| MnSO₄ × 1H₂O | 10 mg/L | 50 | 0,5 | 0,15 | 1 |
| ZnSO₄ × 7H₂O | 1 mg/L | 50 | 0,05 | 0,15 | 1 |
| CuSO₄ × 5H₂O | 0,2 mg/L | 50 | 0,0155 | 0,15 | 1 |
| NiCl₂ × 6H₂O | 0,02 mg/L | 50 | 0,001 | 0,15 | 1 |
| Biotin | 0,2 mg/L | 20 | 0,004 | 0,15 | - |
| Protokatechusäure | 30 mg/L | 20 | 0,06 | 1,5 | 6,5 |
| Glucose | 16 g/L | 200 | 103,2 | 4, 651 | - |

### 5.3 Vorkultur

Ein steriles, verschließbares Reagenzglas wird mit 2 mL Standardmedium befüllt, mit einer Bakterienkultur des Brevibakterium flavum-Stammes DM1730 (Georgi et al. VF (2005) Metab. Eng. A(4): 291-301) von Platte angeimpft (inokuliert) und im Wärmeschrank bei 31°C inkubiert. Mit dieser Vorkultur werden nach 24 h die Schüttelkolben inokuliert. Dazu wird die OD (Optische Dichte) der Vorkultur bei einer Wellenlänge von 600 nm bestimmt und dann auf die gewünschte OD mit sterilem Wasser verdünnt.

### 5.4 Hauptfermentation

Die Fermentation erfolgt zur Bestimmung der Stoffwechselaktivität mit dem RAMOS-System (Fa. HiTec Zang, Herzogenrath). Dieses ahmt einen Bioprozess in einem gewöhnlichen Schüttelkolben nach. Es zeichnet dabei die aus Partialdruckmessungne gewonnen Daten der Sauerstofftransferrate (OTR), der Kohlendioxidtransferrate (CTR) und des Respirationsquotionenten (RQ) auf und stellt sie in der angeschlossenen Rechnereinheit grafisch dar.

Das Flüssigkeitsvolumen der Ramoskolben darf 10 mL nicht überschreiten und zum Animpfen wird 1 % des Volumens veranschlagt. So ergibt sich ein Inokulum von 100 µL, das zu 9,9 mL Medium gegeben wird. Die Kolben werden befüllt, gewogen und im Schüttler befestigt. Nach Dichtigkeitstest und Sauerstoffkalibrierung wird die Messung gestartet. Anhand der Aufzeichnungen des Computers wird der Verlauf der Stoffwechselaktivität verfolgt. Die Messung erfolgt immer als Doppelbestimmung. Nach Beendigung der Fermentation werden OD, Glucose- und Aminosäuregehalt bestimmt.

Die Bakterien im Schwefelsäuremedium wachsen deutlich später an als diejenigen im Standardmedium. Es scheint ein Hemmstoff enthalten zu sein, der für die längere lag-Phase (Anpassungsphase) verantwortlich ist. Die Bakterien müssen ihren Stoffwechsel zunächst an diese Bedingungen adaptieren, bevor sie in die Wachstumsphase eintreten. Dort ist ihre Wachtumsrate jedoch genauso groß, wie die des Standardmediums. Diese Hemmstoffe könnten Furfural und 5-Hydroxymethylfurfural sein, die bei Säurebehandlung von Zuckern entstehen. Sie sind als Fermentationsinhibitoren bekannt. Nach 15 h ist eine Schulter in der Kurve zu erkennen. Dies deutet auf diauxisches Wachstum hin. Das Bakterium stellt hier seinen Stoffwechsel auf weitere C-Quellen um, dadurch bleibt die Stoffwechselaktivität zunächst konstant.

Das Medium aus der oxidativen Vorbehandlung erlaubt gleich frühes Wachsum wie im Standardmedium und auch dieselbe Wachstumsrate. Nach 10 h ist im Kurvenverlauf ebenfalls eine Schulter zu erkennen. Eine zweite tritt nach 14 h auf. Das bedeutet, dass C. glutamicum außer Glucose nach andere C-Quellen metabolisiert, die im Hydrolysat enthalten sind. Das können neben anderen Zuckern aus der Hemicellulose auch Zuckerderivate sein. Eine Vermutung ist Gluconsäure, die bei der Oxidation von Glucose durch Hydrolyse von zunächst gebildetem Glucono-o-lacton entsteht. Gluconsäure ist ein Zwischenprodukt des Stoffwechsels von C.glutamicum und könnte somit aus dem Medium in den Stoffwechselweg eingeschleust werden.

Acetat, das aus der Hemicellulose freigesetzt wird, kann von C. glutamicum co-metabolisiert werden. Das heißt, dass es gleichzeitig mit Glucose verwertet wird, ohne dass der Stoffwechsel umgestellt wird. Acetat bewirkt somit keine Diauxie. Das Citrat aus dem Hydrolysepuffer kann ebenfalls von C. glutamicum verwertet werden. In dem Hydrolysat der Vorbehandlung mit NaOH scheint ein Stoff toxisch für die Bakterien zu sein, da die Stoffwechselaktivität komplett gehemmt ist.

### 5.5 Herstellung von L-Lysin

In Tabelle 4 sind die Ergebnisse der Fermentation angeführt, die unter Verwendung des erfindungsgemäß hergestellten Hydrolysate gefunden wurde.

**Tabelle 4:**

| Vorbehandlung mit | pH-Wert der Vorbehandlung | Glucose nach Hydrolyse [g/L] | Glucose vor Fermentation [g/L] | Lysin nach Fermentation [g/L] |
|---|---|---|---|---|
| Schwefelsäure* | 1 | 2, 93 | 9, 6 | 4, 68 |
| Kaliumperoxid* | 10-11 | 3,42 | 9, 0 | 4,47 |
| Kaliumperoxodisulfat | 1-2 | 4,7 | 11,12 | 6,71 |
| Kaliumhydroxid | 10 | 12,1 | 16, 92 | 6,71 |

| | | | | |
|---|---|---|---|---|
| Weitere Materialien: *20 g Maisstroh, 2 ml Enzym Sonstige: 61 g Weizenstroh, 40 mL Enzym | | | | |

Tabelle 5 enthält die unter Verwendung von nicht erfindungsgemäß hergestellten Hydrolysate erzielten Ergebnisse. Es zeigt sich, dass, obwohl die Fermentationsbrühe Glucose enthält, diese nicht zur Herstellung von L-Lysin metabolisiert wird.

Dieser Umstand beweist, dass zur Herstellung verwertbarer Hydrolysate bestimmte Bedingungen eingehalten werden müssen.

**Tabelle 5:**

| Vorbehandlung mit | pH-Wert der Vorbehandlung | Glucose nach Hydrolyse [g/L] | Glucose vor Fermentation [g/L] | Lysin nach Fermentation |
|---|---|---|---|---|
| Peressigsäure* | 2-3 | 2,35 | 9, 0 | 0 |
| Natriumhydroxid* | 14 | 2,52 | 8, 95 | 0 |
| Wasserstoffperoxid | 3 | 6, 66 | 13,28 | 0 |
| Natriumhypochlorit | 7 | 2,56 | 11,76 | 0 |
| 2-Schritt* | Erst 10, dann 1 | 4, 07 | 14, 84 | 0 |

| | | | | |
|---|---|---|---|---|
| Weitere Materialien: *= 20 g Maisstroh, 2 mL Enzym; Sonstige: 61 g Weizenstroh, 40 mL Enzym | | | | |

## Patentansprüche

1. Verfahren zur Herstellung von zuckerhaltigen Hydrolysaten aus Lignocellulose enthaltenden Materialien, umfassend
a) die Vorbehandlung dieser Materialien mit Peroxodisulfaten, insbesondere Kaliumperoxodisulfat oder Ammoniumperoxodisulfat, in Gegenwart von Wasser, und
b) nach der Abtrennung der wässrigen Phase und Waschen des entstandenden Produkts dessen Behandlung mit einem für die Hydrolyse geeigneten Enzym in Gegenwart von Wasser,
wobei die Hydrolysate als C-Quelle für Fermentationen geeignet sind.

2. Verfahren gemäß Anspruch 1, bei dem man als Lignocellulose enthaltende Materialien Holz von Laub- und Nadelbäume verwendet.

3. Verfahren gemäß Anspruch 1, bei dem als Lignocellulose enthaltende Materialien Stroh einsetzt, ausgewählt aus den Pflanzenarten Mais, Roggen, Weizen, Hafer, Gerste, Sroghum, Raps oder Reis, oder Bagasse.

4. Verfahren gemäß den Ansprüchen 1 bis 3, bei dem man Alkaliperoxodisulfate bei einem pH-Wert von 1 bis 3 einsetzt.

5. Verfahren gemäß den Ansprüchen 1 bis 3, **dadurch gekennzeichnet, dass** die Lignocellulose enthaltenden Materialien Partikelgrößen zwischen 0,5-10 mm, vorzugsweise zwischen 0,5-5 mm, insbesondere zwischen 1-3 mm aufweisen.

6. Verfahren gemäß den Ansprüchen 1 bis 5, an das sich anschließt:
Verfahren zur Herstellung einer organischen Zielsubstanz mit mindestens 3 C-Atomen oder mindestens 2 C-Atomen und einem N-Atom durch Fermentation von diese Substanzen herstellenden Mikroorganismen, wobei man das erfindungegemäß hergestellte zuckerhaltige Hydrolysat als C-Quelle einsetzt.

7. Verfahren gemäß Anspruch 6, bei dem man nach Abschluß der Fermentation die Zielsubstanz isoliert, gegebenenfalls zusammen mit der Gesamtmenge oder Teilen der während der Fermentation gebildeten Biomasse.

8. Verfahren gemäß den Ansprüchen 6 oder 7, bei dem man Zielprodukte herstellt, ausgewählt aus der Gruppe:
organische, gegebenenfalls Hydroxylgruppen tragende Mono-, Di- und Tricarbonsäuren mit 3 bis 10 C-Atomen, proteinogene und nicht-proteinogene L-Aminosäuren, gesättigte und ungesättigte Fettsäuren; Diole mit 3 bis 8 C-Atomen, höherwertigen Alkohole mit 3 oder mehr Hydroxylgruppen, längerkettigen Alkohole mit wenigstens 4 C-Atomen, Vitamine, Provitamine, Ketone mit 3 bis 10 C-Atomen.

9. Verfahren gemäß den Ansprüchen 6 oder 7, bei dem die hergestellten Zielprodukte ausgewählt sind unter L-Aminosäuren und Vitaminen wie insbesondere L-Lysin, L-Methionin, L-Threonin, L-Prolin, L-Isoleucin, L-Homoserin, L-Valin, Pantothensäure und Riboflavin, sowie Propionsäure, Propandiol, Butanol und Aceton.

10. Verfahren gemäß den Ansprüchen 6 oder 7, bei dem die Mikroorganismen ausgewählt sind unter L-Aminosäuren oder Vitamine, überproduzierenden Mikroorganismen, insbesondere unter L-Lysin, L-Methionin, L-Threonin, L-Prolin, L-Isoleucin, L-Homoserin, L-Valin, Propionsäure, Propandiol, Butanol, Aceton und Trehalose überproduzierenden Mikroorganismen.

11. Verfahren gemäß den Ansprüchen 6 oder 7 und 10 bei dem die Mikroorganismen ausgewählt sind unter den Gattungen Corynebacterium, Bacillus, Escherichia, Aspergillus, Lactobacillus und Clostridium, insbesondere unter Stämmen von Corynebacterium glutamicum, Bacillus subtilis, Escherichia coli oder Aspergillus niger.
